# EUROPEAN PATENT SPECIFICATION

(11) **EP 2 321 409 B1**
(45) Date of publication and mention of the grant of the patent: **28.08.2013**
(21) Application number: 09781074.1
(22) Date of filing: 24.07.2009
(51) Int. Cl.: A23B 4/20, A23L 3/3526, C12N 9/24

(54) **NEW ENDOLYSIN PLYP40**
NEUES ENDOLYSIN PLYP40
NOUVELLE ENDOLYSINE PLYP40

(30) Priority: 25.07.2008 DE 102008002972
(43) Date of publication of application: 18.05.2011
(73) Proprietor: Biomérieux S.A., 69280 Marcy L'Etoile (FR); Hyglos Invest GmbH, 82347 Bernried (DE)
(72) Inventor: LOESSNER, Martin, CH-8123 Ebmatingen (CH); SCHMELCHER, Mathias, 86947 Schwabhausen (DE)
(74) Representative: Dehmel, Albrecht
(86) International application number: PCT/EP2009/059606
(87) International publication number: WO 2010/010192

(56) References cited:
- WO-A-2004/004495
- WO-A-2007/093849
- KORNDORFER ET AL: "The Crystal Structure of the Bacteriophage PSA Endolysin Reveals a Unique Fold Responsible for Specific Recognition of Listeria Cell Walls" JOURNAL OF MOLECULAR BIOLOGY, LONDON, GB, vol. 364, no. 4, 16 November 2006 (2006-11-16), pages 678-689, XP005741026 ISSN: 0022-2836
- DATABASE UniProt [Online] 25 November 2008 (2008-11-25), "SubName: Full=Gp26;" XP002546513 retrieved from EBI accession no. UNIPROT:B6D7J9 Database accession no. B6D7J9

## Description

The present invention relates to a polypeptide with an amino acid sequence according to SEQ ID NO:1. The present invention further relates to the nucleic acid molecules comprising a nucleotide sequence coding for the polypeptide, vectors comprising the nucleic acid molecules, and host cells for the expression of the polypeptide. Moreover, the present invention relates to the use of the polypeptide as a human medical, veterinary medical or diagnostic substance, as an antimicrobial agent in food, in cosmetics, as disinfecting agent or in the environmental field.

Listeria are widespread human and animal pathogenic bacteria in the food sector, which elicit the disease pattern of listeriosis. Food products like fish, meat and dairy products are frequently contaminated with listeria. The genus *Listeria* comprises 6 distinct species with 16 different serotypes. In detail these are *L. monocytogenes* having the serotypes 1/2a, 1/2b, 1/2c, 3a, 3b, 3c, 4a, 4ab, 4b, 4c, 4d, 4e, 7; *L. innocua* having the serotypes 3, 6a, 6b, 4ab, U/S; *L. ivanovii* having the serotype 5; *L. seeligeri* having the serotypes 1/2a, 1/2b, 1/2c, 4b, 4c, 4d, 6b; *L. welshimeri* having the serotypes 1/2a, 4c, 6a, 6b,U/S, and *L. grayi* having the serotype Grayi. The two species *L. monocytogenes* and *L. ivanovii* are considered to be pathogenic. A third species, *L. seeligeri*, is regarded to be nonpathogenic, however there is one case known, in which *L. seeligeri* caused meningitis in a human being. The remaining species are considered to be nonpathogenic. About 90% of the listerioses are attributed to *L*. *monocytogenes* serovar 1/2a, 1/2b, and 4b (Wing EJ & Gregory SH, 2002, Listeria monocytogenes: Clinical and Experimental Update, J Infect Diseases 185 (Suppl 1): S 18-S24).

In fact, listeriosis is a rare disease, but it has to be taken very seriously because of the severity of the disease and the high mortality rate. Although only a minimal percentage of the food related diseases is induced by *listeria* (about 1% in the USA), almost 30% of the annual illnesses with fatal outcome, which are caused by food pathogens, are assigned to this pathogen. Affected are primarily immunosuppressed persons, e.g. elderly people, diabetics, and persons suffering from cancer and/or AIDS. Pregnant women and the unborn child constitute about 25% of all cases of listeriosis patients. Based on their ability to cross the blood brain barrier or the placental barrier, listeria may cause meningitis, encephalitis, abortions, and stillbirths (Wing EJ & Gregory SH, 2002, Listeria monocytogenes: Clinical and Experimental Update, J Infect Deseases 185 (Suppl 1): S18-S24; Doyle ME, 2001, Virulence Characteristics of Listeria monocytogenes, Food Research Institute, October 2001).

Listeria are very well adapted to the survival in the environment during the food production. They are tolerant to weak acids and are capable of reproducing at relatively high salt concentrations and at temperatures from 1°C to 45°C. The main source of infection are articles of food, in particular those which are not heat-treated prior to consumption, such as many dairy products, smoked fish, salted fish, frozen seafood, meat products, salads, and also at an increasing rate convenience products ("ready-to-eat"-products, especially meat products). The contamination by listeria frequently takes place during the processing of food (removal from the cooking container, slicing, decorating, packaging, etc.). In food products, which are produced with the aid of starter cultures and are not heat-treated (e.g., raw milk cheese, salami), a contamination can also take place through the starter cultures or the raw materials themselves or also during ripening and storage. While there is a zero tolerance for *L. monoocytogenes* in ready-to-eat food in the USA, in many European countries, or Canada as well, a contamination with listeria of up to 100 CFU (colony forming units)/g food is permitted for specific food products. Nevertheless, the food products have to be tested for contamination by listeria in all cases. Many of these food products, e.g. seafood, smoked salmon, dairy products or even prefabricated raw food products, have only a limited shelf life. This frequently results in cost-intensive product recalls, if a listeria contamination and a contamination above the permitted limit, respectively, were detected in these products after delivery.

For this reason there is a great interest in providing methods for the detection as well as for decontamination of listeria. Furthermore, uses of antimicrobial substances are important to prevent the growth of listeria on the one hand and to kill already present listeria on the other hand.

EP0781349 describes inter alia the *Listeria* phage lysin, Ply511, from the phage A511 that can be used for the aforementioned applications. Based on its broad host range against a multitude of *Listeria* serovars, Ply511 is very well suited, however, it exhibits a relatively low stability, which is an obstacle especially to its application in food products. Thus, Turner et al. (2007, Syst. And Appl. Microbiol., 30, 58-67) point out proteolysis problems in the expression of Ply511 in lactobacilli for the potential use in food products.

EP 1 531 692 B1 describes the *Listeria*-phage P100 and the *Listeria*-phage lysin PlyP100 coded by it.

Both Ply511 and PlyP100 have an optimum activity at a weakly alkaline pH, whereas an optimum activity in the neutral and weakly acidic pH range would be important in many applications.

It is therefore the object of the present invention to provide endolysins against listeria, which have a higher stability as well as a higher activity in the weakly acidic range.

This object is solved by the subject-matter defined in the claims.

The following figures serve to illustrate the invention.

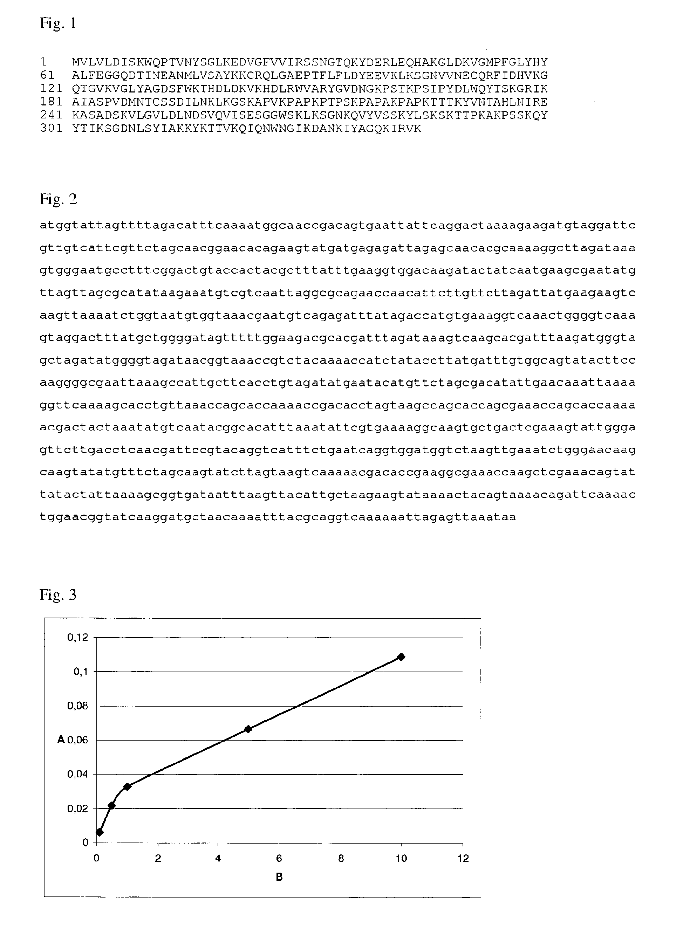

Figure 1 shows the amino acid sequence of the endolysin PlyP40 (SEQ ID NO: 1) according to the present invention.
Figure 2 shows the nucleotide sequence (SEQ ID NO:2) coding the endolysin PlyP40 according to the present invention.
Figure 3 shows in a graph the concentration dependence of the lysis activity of endolysin PlyP40 according to the present invention against *L. innocua* S 1147 (SV 6b). The change in the absorbance per minute (A) was determined as a function of the concentration of PlyP40 (B) in µg/ml.

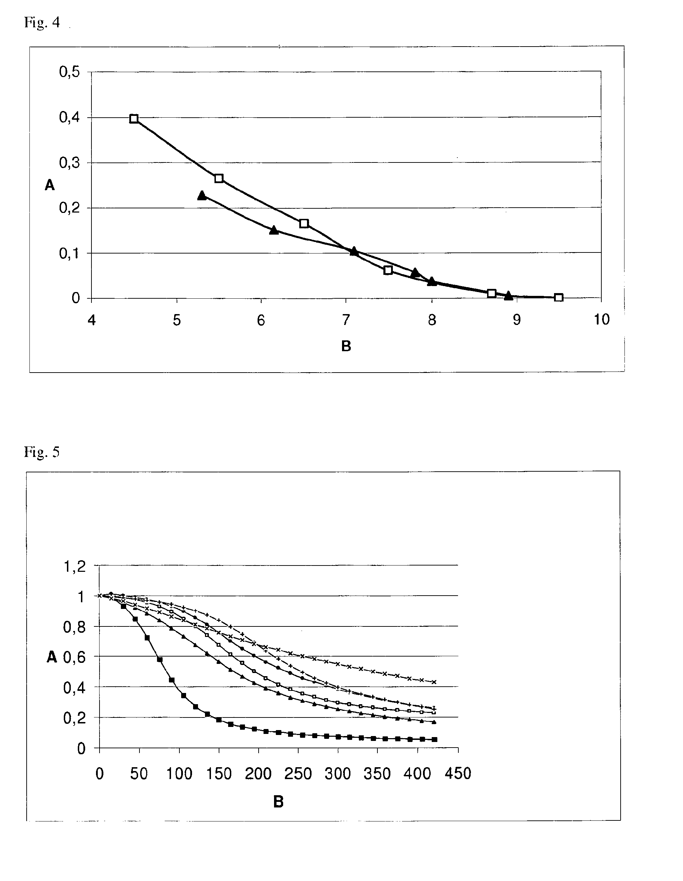

Figure 4 shows in a graph the pH dependence of the lysis activity of endolysin PlyP40 according to the present invention against *L. innocua* S1147 (SV 6b). The change in the absorbance per minute (A) was determined as a function of the pH value (B) for 12.8 µg of the endolysin PlyP40 in 1x PBST in the pH range from 5 to 9 (A) and for 12.8 µg of the endolysin PlyP40 in 50 mM citrate, 50 mM NaH₂PO₄, 50 mM borate in the pH-range from 4.5 to 9.5 (□).
Figure 5 shows in a graph the activity of the endolysin PlyP40 of the present invention against different *Listeria* strains. Substrate cells of the *Listeria* strains *L. monocytogenes* 1442 SV1/2a (■), *L. monocytogenes* 1042 SV 4b (□), *L. monocytogenes* 1019 SV 4c (●), *L. monocytogenes* 1001 SV 1/2 c (▲), *L. innocua* 2011 SV 6a (+), and *L. welshimeri* 50146 SV 6a (x) were normalized to an initial OD₆₀₀ of 1.0. The normalized OD₆₀₀ (A) was traced at a temperature of 30°C in 1x PBS, pH 8.0, at an initial PlyP40 concentration of 200 pmole/ml as a function of the time (B) in seconds.

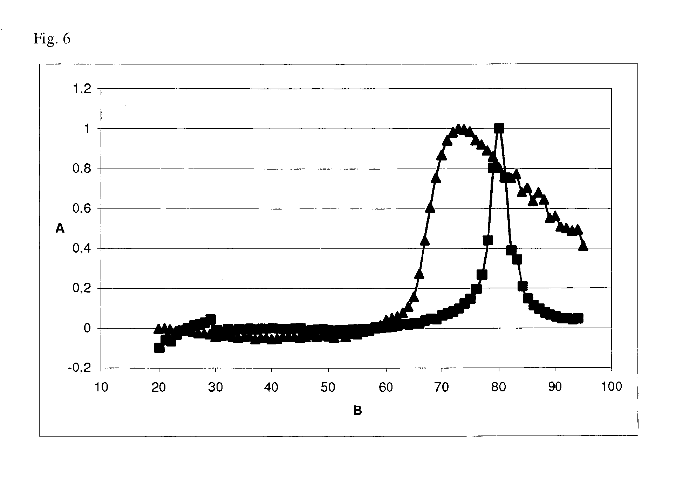

Figure 6 shows in a graph the analysis of a thermal stability test of the endolysins PlyP40 and Ply511. Endolysin solutions of PlyP40 (■) and Ply511 (▲) were heated in the photometer. In this process, the increase in the protein aggregation, which corresponds to an increase of the absorbance (A) at a wavelength of 360 nm, is traced as a function of the temperature (B) in degrees Celsius.

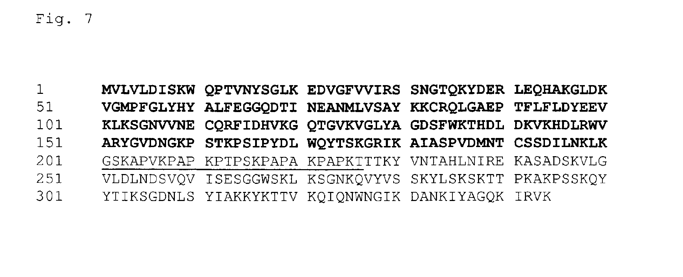

Figure 7 shows the amino acid sequence of the endolysin PlyP40 (SEQ ID NO: 1) according to the present invention. The N-terminal amino acids in bold type at the positions from 1 to 200 represent the enzymatically active domain (EAD). The cell binding domain (CBD) of PlyP40 comprises the C-terminal located amino acids from 227 to 344. The underlined amino acid sequences represent a 26 amino acid linker from position 201 to 226.

The term "listeria" as used herein denotes all bacteria, which are assigned to the genus *Listeria.* In particular, the term listeria encompasses the species *L. monocytogenes* having the serotypes 1/2a, 1/2b, 1/2c, 3a, 3b, 3c, 4a, 4ab, 4b, 4c, 4d, 4e, 7; *L. innocua* having the serotypes 3, 6a, 6b, 4ab, U/S; *L. ivanovii* having the serotype 5; *L. seeligeri* having the serotypes 1/2a, 1/2b, 1/2c, 4b, 4c, 4d, 6b; *L. welshimeri* having the serotypes 1/2a, 4c, 6a, 6b,U/S, and *L. grayi* having the serotype Grayi.

The term "endolysin", as used herein, denotes enzymes that are naturally coded by bacteriophages and are produced by them at the end of their host cycle to lyse the host cell and thereby release the offspring phages. Endolysins are comprised of at least one enzymatically active domain (EAD) and a non-enzymatically active cell binding domain (CBD). The EADs can exhibit different enzymatic activities such as, e.g. N-acetyl-muramoyl-L-alanin amidase (amidase, e.g., Ami_2, Ami_5), (endo)-peptidase (e.g., CHAP), transglycosylase, glycosyl hydrolase, (N-acetyl)-muramidase (lysozyme), N-acetyl-glucosaminidase.

The term bacterial "cell wall", as used herein, denotes all components that form the outer cell enclosure of the bacteria und thus guarantee their integrity. In particular, this refers to the peptidoglycan, the outer membrane of the gram-negative bacteria with the lipopolysaccharide, the bacterial cell membrane, but also to additional layers deposited on the peptidoglycan, like capsules, slimes or outer protein layers.

The term "domain" or "protein domain", as used herein, denotes a portion of an amino acid sequence that either has a specific functional and/or structural property. On the basis of amino acid sequence homologies, domains can frequently be predicted by employing appropriate computer programs that compare the amino acid sequences in freely available databases with known domains, e.g., Conserved Domain Database (CDD) at the NCBI (Marchler-Bauer et al., 2005, Nucleic Acids Res. 33, D192-6), Pfam (Finn et al., 2006, Nucleic Acids Research 34, D247-D251), or SMART (Schultz et al., 1998, Proc. Natl. Acad. Sci. USA 95, 5857-5864, Letunic et al., 2006, Nucleic Acids Res 34, D257-D260).

The term "domain linker", as used herein, denotes an amino acid sequence functioning to connect single protein domains with one another. As a rule, domain linkers form no or only few regular secondary structures like α-helices or β-sheets and can occupy different conformations within the respective structural context. Properties of linker sequences as well as methods to detect those are described in the prior art (George & Heringa, 2003, Protein Engineering, 15, 871-879, Bae et al., 2005, Bioinformatics, 21, 2264-2270).

The term "CBD", as used herein, relates to polypeptide fragments, wherein the respective amino acid sequence corresponds to a portion in endolysins. Said portion is responsible for the binding of the endolysins to the listeria cell wall. Said polypeptide fragments are not enzymatically active. The CBD may also be present as a gene fusion with a spacer molecule (GFP, MBP, biotinylation domains) with and without an affinity tag (His-Tag, Strep-Tag, Avi-Tag, biotinylation domains) or also as a gene fusion only with affinity tag (His-Tag, Strep-Tag, Avi-Tag, biotinylation domains).

The term "EAD" as used herein refers to the enzymatically active domain of a peptidoglycan lysing enzyme which is responsible for hydrolysis of the bacterial peptidoglycan. It contains at least one of the enzymatic activities described for a peptidoglycan lysing enzyme. The term EAD as used herein describes a segment within a polypeptide chain which is derived from a naturally occurring peptidoglycan lysing enzyme.

The term "wild type" or "wt", as used herein, denotes the amino acid sequence of the endolysin PlyP40 from the phage P40 as specified in SEQ ID NO: 1. The term denotes also the nucleic acid sequence coding the amino acid sequence according to SEQ ID NO: 1.The nucleic acid sequence that codes the endolysin PlyP40 and has been isolated from the phage P40 is specified in SEQ ID NO: 2. The term also encompasses the nucleic acid sequence which contains different codons for single amino acids than those specified in SEQ ID NO: 2, but codes the same amino acid sequence due to the degenerated code.

The term "polypeptide" or "protein", as used herein, denotes peptides consisting of at least 8 amino acids. The polypeptides can be pharmacologically or immunologically active polypeptides, polypeptides used for diagnostic purposes, or polypeptides used as antimicrobial agent.

The term "protease", as used herein, denotes an enzyme that is capable of hydrolytically cleave peptide bonds of proteins and/or peptides. The term encompasses proteases, which cleave single amino acids from the amino or the carboxy terminus as well as proteinases which cleave within a protein or polypeptide.

The term "variants", as used herein, means that a polypeptide has an altered amino acid sequence in comparison to the wild type sequences. The alterations can involve modifications, substitutions, mutations, deletions, additions, and insertions.

The term "mutation", as used herein, means an alteration of the starting amino acid sequence. Here individual or several immediately consecutive amino acids or amino acids interrupted by non-modified amino acids can be deleted (deletion), added (insertion or addition), or substituted by other amino acids (substitution). The term also encompasses a combination of the individual mentioned alterations. The term encompasses also the N- or C-terminal fusion of a protein or peptide tag.

The term "modification", as used herein, can be used synonymously with "mutation". However, the term "modification", as used herein, also encompasses chemical modifications of the amino acids like e.g., biotinylation, acetylation, chemical modification of the amino-, SH-, or carboxyl groups.

The term "deletion", as used herein, means the removal of 1, 2 or more amino acids from the respective starting sequence.

The term "insertion" or "addition", as used herein, means the removal of 1, 2 or more amino acids from the respective starting sequence.

The term "substitution", as used herein, means the exchange of an amino acid located at a certain position for a different one.

The present invention therefore relates to polypeptides possessing the amino acid sequence according to SEQ ID NO: 1.

The endolysin PlyP40 has a length of 344 amino acids in its wild type form. It possesses two functional domains that have only a minimal homology with other known endolysins. The N-terminal amino acids at the positions from 1 to 200 represent the enzymatically active domain (EAD). The cell binding domain (CBD) of PlyP40 comprises the C-terminal located amino acids from 227 to 344. The two domains are connected by a 26 amino acid linker from position 201 to 226.

The present invention further relates to the polypeptides according to the invention comprising modifications. The present invention further relates to the nucleotide sequences coding the polypeptides according to the present invention. The modified polypeptides exhibit the lytic activity of the Wt-PlyP40 endolysin, wherein the activity can be higher, the same or lower, but is not completely lost. The activity is measured with assays known to a person skilled in the art, e.g., the plate lysis assay or the liquid lysis assay.

The modifications can be mutations, in particular deletions, insertions or additions, substitutions or combinations thereof.

Preferably, the deletions introduced into the amino acid sequence according to SEQ ID NO: 1 of the naturally occurring endolysin PlyP40 can shorten the amino acid sequence such that the activity of the protein is not lost. For example, the protease cleavage sites can be removed through the introduced deletions.

The deletions may involve one or several amino acids. When several amino acids are deleted, then the deleted amino acids may be immediately adjacent to each other. Moreover, single deleted amino acids or regions with several deleted amino acids may be separated from each other by one or several non-deleted amino acids. Therefore, one or several deletions may be inserted in the starting sequence of the endolysin PlyP40 according to SEQ ID NO: 1.

Preferably, the substitutions introduced into the amino acid sequence according to SEQ ID NO: 1 of the naturally occurring endolysin PlyP40 can change the amino acid sequence such that the activity of the protein is not lost. For instance, the protease cleavage sites may be altered through the introduced substitutions in such a way that the protease which is specific for the cleavage site does no longer cleave the endolysin.

The substitutions may involve one or several amino acids. When several amino acids are substituted, then the substituted amino acids may be immediately adjacent to each other. Moreover, single substituted amino acids or regions with several substituted amino acids may be separated from each other by one or several non-substituted amino acids. Therefore, one or several substitutions can be inserted in the starting sequence of the endolysin PlyP40 according to SEQ ID NO: 1.

Modifications such as N- or C-terminal tags or chemical modifications of single amino acids may be added to facilitate the production of the proteins (e.g., His-tag or Strep-tag for easier purification), to improve its utilization (e.g., Strep-tag, Avi-tag, JS-tag or chemical biotinylation for the immobilization on surfaces that possess streptavidin or avidin), or enhance solubility or stability (e.g., PEGylation). Furthermore, the modifications can comprise N- or C-terminal HA-tags, Myc-tags or GST-tags. All above mentioned tag-sequences are well known to people skilled in the art. The sequences can be obtained from literature or commercially available vectors.

All of the modified PlyP40 endolysins according to the invention exhibit a lysis activity that is identical or comparable to the naturally occurring PlyP40 endolysin. Furthermore, the above described modifications exhibit positive effects that are beneficial for a commercial application of the endolysins. Such positive effects may involve an enhanced protease stability, thermal stability or stability against chemical denaturing agents. In addition, the stabilization can lead to a higher expression rate, solubility or a longer shelf life. The positive effect may also be expressed by an enhanced activity.

The present invention further relates to polypeptide fragments of the endolysin PlyP40 having the property to bind to the cell wall of listeria, wherein the polypeptide fragments do not exhibit any enzymatically active cell wall hydrolysing regions anymore. Furthermore, the invention relates to the nucleic acid sequences coding for the polypeptide fragments according to the invention. The polypeptide fragments according to the invention are referred to in the following also as "cell wall binding domains" (CBD).

Preferably, the polypeptide fragments according to the invention exhibit an amino acid sequence (referring to the full-length sequence according to SEQ ID NO:1) from about position 227 to 344 as denoted in SEQ ID NO:4. Preferably, the invention relates furthermore to nucleic acid molecules encoding the described preferred polypeptide fragments.

Especially the CBD may be coupled to low molecular substances, *e.g*., biotin. It may be chemically introduced into the CBD or by fusion of the CBD with a polypeptide, in which biotin is introduced *in vivo* or *in vitro* using another protein. Such polypeptides are, *e.g.,* biotinylation domains, i.e., regions in naturally occurring polypeptides, which are biotinylated. Such biotinylation domains are exhibited, *e.g*., by the oxalacetate decarboxylase of *Klebsiella* (US 5,252,466 and EP 0511747), the *Salmonella typhimurium* oxalacetate decarboxylase, the *Propionibacterium shermanii* transcarboxylase subunit, the biotin carboxyl carrier protein of the *Escherichia coli* acetyl-CoA carboxylase, the *Saccharomyces cerevisiae* pyruvate carboxylase or the *Saccharomyces cer-evisiae* acetyl-CoA carboxylase. Such a polypeptide may, however, also be the Avi-Tag (avidity-patents US 5,932,433, US 5,874,239, and US 5,723,584). Furthermore, a biotin may be chemically specifically coupled to a group by fusion with a polypeptide which carries said group, which is not or seldom - but hardly accessible - present in the protein (*e.g*., cysteine). Furthermore, instead of biotin, the so-called Strep-Tag (Skerra, A. & Schmidt, T. G. M. Biomolecular Engineering 16 (1999), 79-86, US 5,506,121) may be used, which is a short amino acid sequence and binds to streptavidin. Furthermore, the His-Tag may be used. It is also possible to combine different tags and in such a way to use the different binding affinities of the different tags, *e.g*., Strep-Tag and His-Tag, or biotinylation domain and His-Tag. The biotinylation domains as well as the Avi-Tag, the Strep-Tag as well as the His-Tag are preferably coupled to the CBD using DNA-recombination technology. Preferably, the fusion protein consists of the biotinylation domain of the oxalacetate decarboxylase from *Klebsiella* or the Avi-Tag, the Strep-Tag or the His-Tag, which are bound to the N-terminal end of the CBD at their C-terminal end. Such a fusion, however, may also be one of the above-mentioned tags, with whose C-terminal end the N-terminus of another protein, which is used as a kind of "spacer molecule", is coupled, *e.g*., GFP or maltose binding protein. In this case, the CBD may be coupled via its N-terminal end to the C-terminal end of said other protein.

The CBDs according to the invention may be used for methods for enrichment, removal, and detection of listeria as described in the state of the art.

The present invention further relates to polypeptide fragments of the endolysin PlyP40 having the property to enzymatically hydrolyse the cell wall of Listeria. Furthermore, the invention relates to the nucleic acid sequences coding for the polypeptide fragments according to the invention. The polypeptide fragments according to the invention are referred to in the following also as "enzymatically active domains" (EAD).

Preferably, the polypeptide fragments according to the invention exhibit an amino acid sequence (referring to the full-length sequence according to SEQ ID NO:1) from about position 1 to 200 as denoted in SEQ ID NO:3. Preferably, the invention relates furthermore to nucleic acid molecules encoding the described preferred polypeptide fragments.

Preferably, the invention further relates to nucleic acid molecules comprising nucleotide sequences which code the described modified polypeptides according to the invention. Preferably, a nucleic acid molecule according to the invention comprises a nucleotide sequence according to SEQ ID NO: 2.

The present invention further relates to vectors comprising the nucleic acid molecules according to the invention.

The present invention further relates to appropriate host cells for the expression of the polypeptides according to the invention. Preferably, a suitable host cell for the expression of the polypeptides according to the invention comprises a nucleic acid molecule according to the invention or a vector according to the invention. Preferably, a suitable host cell for the expression of the polypeptide according to the invention is transformed with a nucleic acid molecule according to the invention.

The present invention further relates to the use of the proteins according to the invention as human medical, veterinary medical, or diagnostic substance, as an antimicrobial agent in food or in cosmetics, or as disinfecting agent.

The present invention further relates to a pharmaceutical comprising a polypeptide according to the present invention. The present invention further relates to a pharmaceutical composition comprising the polypeptide according to the present invention. Preferably, a pharmaceutical composition according to the present invention may additionally comprise a pharmaceutically acceptable buffer, a pharmaceutically acceptable diluting agent, or a pharmaceutically acceptable carrier. Moreover, a pharmaceutical composition according to the present invention may contain suitable stabilizing agents, flavors or other suitable reagents.

A further aspect of the present invention relates to the polypeptides according to the invention for the use as human medical, veterinary medical or diagnostic substance for therapy and/or prevention of diseases that are caused by listeria or for the diagnosis of listeria contaminations.

Diseases that are caused by listeria comprise, among others, listeriosis, gastroenteritis, meningitis, encephalitis, sepsis, local wound infections caused by smear infections and inflammations of conjunctiva and cornea.

In a further aspect of the present invention, the polypeptide according to the invention is used in a method for the treatment and/or prophylaxis of infections, in particular of infections that are caused by *Listeria.* In particular, this *Listeria* infection can be an infection caused by L. *monozytogenes,* preferably by *L. monocytogenes* with the serotypes 1/2a, 1/2b, 1/2c, 3a, 3b, 3c, 4a, 4ab, 4b, 4c, 4d, 4e, 7, especially by *L. monocytogenes* 1442 SV1/2a, *L*. *monocytogenes* 1042 SV 4b, *L. monocytogenes* 1019 SV 4c and/or *L. monocytogenes* 1001 SV 1/2 c. In addition, this infection can be a *Listeria*-infection caused by *L. innocua,* preferably by *L. innocua* with the serotypes 3, 6a, 6b, 4ab, U/S, especially by *L. innocua* 2011 SV 6a. The patient can be a human patient or an animal, in particular animals, which are used in animal husbandry and/or in dairy farming such as ruminants (e.g., cattle, cows, sheep or goats), pigs, horses, poultry, captive wild birds, rabbits, or predators. The method comprises the application of the polypeptides of the present invention in an adequate amount at the site of infection or at a site that is treated prophylactically against an infection.

In a further preferred embodiment, a polypeptide according to the present invention is used in a method for the treatment and/or prophylaxis of gastroenteritis, in particular, gastroenteritis caused by *Listeria.*

In a further preferred embodiment, a polypeptide according to the present invention is used in a method for the treatment and/or prophylaxis of listeriosis, meningitis, encephalitis, sepsis, as well as local wound infections caused by smear infections and inflammations of conjunctiva and cornea, which are caused especially by Listeria.

In a further preferred embodiment, a polypeptide according to the present invention is used in a method for the treatment and/or the prophylaxis of the above mentioned diseases during prenatal care.

In a particularly preferred embodiment, a polypeptide of the present invention is used for the medical treatment when the infection to be treated or prevented, has been caused by a resistant *Listeria* strain. Moreover, a polypeptide of the present invention can be used in methods for the treatment of infections through administration in combination with conventional antibacterial active substances, such as antibiotics, other enzymes, e.g., endolysins etc.

The dosage and the type of administration used in a method of treatment and/or prophylaxis of the aforementioned diseases depends on the specific disease and also the site of the infection to be treated. For instance, in particular embodiments of the present invention the type of administration can be an oral, topical, parenteral, intravenous, rectal, or any other type of administration. For the application of a polypeptide of the present invention at the site of infection (or the site at risk of being infected), a polypeptide of the present invention may be formulated in a manner such that the polypeptide is protected from environmental influences like proteases, oxidation, or an immune response etc.

Therefore, a polypeptide of the present invention may be present in a capsule, in a dragee, in a pill, in a suppository, in an injectable solution, or in any other medically suitable galenic formulation. In some embodiments of the present invention, this galenic formulation can contain additionally suitable carriers, stabilizers, flavors, buffers or other suitable reagents.

For instance, for topical applications a polypeptide of the present invention can be administered in the form of a lotion or a plaster.

A suppository formulation may be provided for the treatment of the intestine. Alternatively, an oral administration may be considered. In this case, the polypeptide of the present invention has to be protected from the influences of the gastrointestinal environment until it has reached the site of infection. For example, this can be accomplished through the use of bacteria as carriers, which survive the initial steps of digestion in the stomach and secrete a polypeptide of the present invention later on in the intestinal environment.

All medicinal uses are based on the effect of the polypeptide of the present invention to specifically and immediately lyse *Listeria*-bacteria when coming into contact with the bacteria. This has an immediate impact on the health status of the treated patient through the reduction of the pathogenic bacteria and bacterial load and the simultaneous support of the immune system. For this purpose, the same galenic formulations can be used such as those that are used in conventional medications for these applications.

In a further aspect, the polypeptides of the present invention are a constituent part of a cosmetic composition. For example, a cosmetic composition according to the invention can be used to inhibit or to prevent irritations caused through an infection of the skin by *Listeria* bacteria. A cosmetic composition according to the invention preferably contains a sufficient amount of polypeptides according to the invention in order to lyse already existing and/or freshly colonizing *Listeria* bacteria.

A further aspect of the present invention relates to the use of the polypeptides according to the invention and/or host cells as an antimicrobial substance in food such as dairy products, smoked fish, salted fish, frozen seafood, meat products, salads, and convenience products ("ready-to-eat"-products, especially meat products and ready-made raw food products)

A further aspect of the present invention relates to the use of the polypeptides according to the invention as an antimicrobial substance in food processing devices, in food processing facilities, on surface areas that come into contact with food such as shelves, on containers and in facilities that are used for the storage or the processing of food, and in all other situations, where *Listeria* bacteria may infest potential food materials. In this context, the polypeptides according to the invention can be used alone or in combination with different antimicrobial substances like disinfecting agents, antibiotics or enzymes such as, for example, different endolysins.

The polypeptides according to the invention can be introduced into or applied to food products and/or at various technical locations within food processing facilities through a multitude of means like for example by admixture of the polypeptides according to the invention to food products, by spraying of the polypeptides according to the invention on facility devices and/or by directly applying the polypeptides according to the invention onto facility devices.

A further aspect of the invention relates to the use of the polypeptides according to the invention in the diagnosis and the detection, respectively, of listeria contaminations in medicine, food industry and food analysis, livestock breeding, and drinking water analysis or environmental analysis.

Listeria contaminations can be detected with the aid of the polypeptides of the present invention in miscellaneous samples like for example in aqueous solutions and mixtures of water and organic solvents, foods, media, blood, blood products, plasma, serum, urine, stool samples, protein solutions, water/ethanol mixtures as well as in solutions in which nonaqueous solid substances to be assayed or isolated, respectively, are dissolved, such as, for example, proteins, DNA, RNA, sugar, salts, food, food-media homogenates, pharmaceuticals, vaccines, organic and inorganic chemicals (e.g., NaCl, MgCl₂, purines, pyrimidines, etc.).

The following examples illustrate the invention and are not to be considered to limit the scope of the invention. Unless stated otherwise, molecular biological standard methods have been used, such as described e.g. by Sambrook et al., 1989, Molecular cloning: A Laboratory Manual, 2nd edition, Cold Spring Harbor Laboratory Press, Cold Spring Harbor, New York.

### Example 1: Concentration dependence of the lysis activity of endolysin PlyP40 against L. innocua S1147 (SV 6b).

*L. innocua* S1147 (SV 6b) cells were employed as a substrate in a photometrical lysis assay for the determination of the concentration dependence of the lysis activity of endolysin PlyP40. For this purpose, *L. innocua*-cells were suspended in PBS buffer with 0,05% Tween 20 at pH 8.0 (1 x TBST) and thawed. The change in absorption per minute as an indicator of the lysis activity in dependence of the PlyP40 concentration was determined by adding 1 mM DNase and various amounts of endolysin PlyP40 in a total volume of 1 ml. As a result, a change in absorbance d_{abs}/min per µg of protein in the linear range of 0.0294 was determined, so that a definite activity of the endolysin PlyP40 could be demonstrated.

### Example 2: pH dependence of the lysis activity of endolysin PlyP40 against L. innocua S1147 (SV 6b).

To examine the pH dependence of the lysis activity of the endolysin PlyP40, *L*. *innocua* S1147 (SV 6b) cells were employed as a substrate in a photometrical lysis assay. For this purpose, frozen *L*. *innocua* cells were suspended either in 1x PBS buffers (pH 5-9) or in 50 mM citrate, 50 mM NaH₂PO₄, 50 mM borate buffers (pH 4.5 to 9.5) and thawed. Subsequently, the substrate cell suspensions were mixed with 12.8 µg of endolysin PlyP40 each. The change in the absorption per minute was determined in the photometer as an indicator of the lysis activity. It was found that the maximal lysis activity in both buffer solutions is always found at the lowest pH value examined. A steadily decreasing lysis activity was observed with an increasing pH value, where at a pH greater than 8 only a very low or no lysis activity at all was observed. Hence, the optimum of lysis of endolysin PlyP40 is clearly in the acidic range and thus differs from the optima of lysis of the endolysins Ply511 and PlyP 100.

### Example 3: Lysis activity of endolysin PlyP40 against various Listeria strains.

The lytic activity of endolysin PlyP40 against the Listeria strains L. monocytogenes 1442 SV1/2a, L. monocytogenes 1042 SV 4b, L. monocytogenes 1019 SV 4c, L. monocytogenes 1001 SV 1/2 c, L. innocua 2011 SV 6a, and L. welshimeri 50146 SV 6a was evaluated in a photometric lysis assay. The substrate cells of the individual Listeria strains were added as a starting material at an initial OD600 between 1.0 and 1.5 (normalized to 1.0) in 1x PBS buffer at pH 8 in a total volume of 1 ml. PlyP40 was added at a concentration of 200 pmol/ml at the point in time t=0 and the change in the optical density was traced for several minutes as an indictor of the lysis activity of endolysin PlyP40. The assays were run at 30°C. A high lysis activity of the endolysin PlyP40 was determined for all L. monocytogenes strains from different serovar groups as well as for L. innocua, whereas the lysis activity against L. welshimeri was clearly less pronounced.

### Example 4: Comparison of the thermal stability of the two endolysins PlyP40 and Ply511.

For the test of the thermal stability 100 µg each of endolysin PlyP40 and Ply511 in 25 mM Na-phosphate, 100 mM NaCl, pH 8.0, were placed in a stirrable quartz cuvette (volume 1 ml). The increase in the optical density, which occurs at rising temperatures due to an altered light scattering, caused by the aggregation of the proteins, was traced during heating from 20 to 90 °C. A heating rate of 1°C/min was used for heating and the measurement of the optical density was carried out in the photometer at a wavelength of 360 nm. Melting points of 80°C for endolysin PlyP40 and of 68°C for endolysin Ply511 were determined by means of the thermal stability assay.

### Example 5: Binding of GFP-tagged CBDs from different Listeria endolysins to the cell wall of different Listeria

Late log phase cells of several Listeria strains were resuspended in PBST (50 mM NaH2PO4, 120 mM NaCl, PH 8,0, 0,01% Tween 20) and incubated with an excess of fusion proteins of green fluorescence protein (HGFP) and CBDs of different listeria binding proteins which are known in the art and described in Korndörfer et al. (2006: The Crystal Structure of the Bacteriophage PSA Endolysin Reveals a Unique Fold Responsible for Specific Recognition of Listeria Cell Walls. J. Mol. Bio. 364: 678-689) and Loessner et al. (2002, Mol Microbiol. 44, 335-349).. The respective fusion proteins are incubated for 5 min at room temperature. After washing twice with TBST buffer, the cells were prepared for fluorescence microscopy, using an Axioplan microscope (Carl Zeiss). Pictures of green labeled cells were obtained with a filer set with excitation 450-490 nm, beamsplitter 510 nm, and emission 520 nm. HGFP-CBD-P40 containing amino acid 201 to 344 from SEQ ID NO:1 was C-terminally fused to HGFP as described in Loessner et al., 2002, Mol Microbiol. 44, 335-349. The results of the binding assays are given in table 1 below.

**Table 1: Binding of GFP-tagged CBDs from different Listeria phage endolysins to the cell wall of Listeria cells from different species and serovars (++ strong, + weak, (+) very weak, - no binding).**

| **Species** | **WLSC code** | **Source** | **Serovar** | **Binding of CBD** | | | | | | | |
|---|---|---|---|---|---|---|---|---|---|---|---|
| | | | | **118** | **006** | **500** | **PSA** | **P35** | **511** | **P40** | **025** |
| L. monocytogenes | EGDe | J. Kreft | 1/2a | ++ | + | - | - | ++ | ++ | ++ | - |
| L. monocytogenes | 10403S | D. Portnoy | 1/2a | ++ | + | - | - | ++ | + | ++ | - |
| L. monocytogenes | 1442 | Food | 1/2a | ++ | ++ | - | - | - | ++ | ++ | - |
| L. monocytogenes | 1066 | SLCC 8800 | 1/2b | ++ | + | - | - | ++ | + | ++ | - |
| L. monocytogenes | 1001 | ATCC 19112 | 1/2c | ++ | + | - | - | ++ | + | ++ | - |
| L. seeligeri | 4007 | ATCC 35967 | 1/2b | ++ | + | - | - | ++ | + | ++ | - |
| L. welshimeri | 50149 | SLCC 5877 | 1/2b | ++ | ++ | - | - | + | ++ | ++ | - |
| L. monocytogenes | 1485 | soft cheese | 3a | + | (+) | - | - | + | + | ++ | - |
| L. monocytogenes | 1031 | SLCC1694 | 3b | + | - | - | - | ++ | + | ++ | - |
| L. monocytogenes | 1032 | SLCC 2479 | 3c | + | (+) | - | - | ++ | + | ++ | - |
| L. seeligeri | 40127 | SLCC 8604 | 3b | + | - | - | - | ++ | ++ | ++ | - |
| L. monocytogenes | 1034 | SLCC 2482 | "7" | + | (+) | - | - | - | + | ++ | - |
| L. monocytogenes | 1020 | ATCC 19114 | 4a | - | + | ++ | ++ | ++ | ++ | ++ | ++ |
| L. monocytogenes | 1042 | ATCC 23074 | 4b | - | - | ++ | ++ | - | + | ++ | - |
| L. monocytogenes | ScottA | J. Jay | 4b | - | - | ++ | ++ | - | + | ++ | - |
| L. monocytogenes | 1019 | ATCC 19116 | 4c | - | - | ++ | ++ | ++ | + | + | - |
| L. monocytogenes | 1033 | ATCC 19117 | 4d | - | - | ++ | ++ | + | ++ | ++ | - |
| L. monocytogenes | 1018 | ATCC 19118 | 4e | - | - | ++ | + | (+) | ++ | ++ | - |
| L. ivanovii | 3009 | SLCC 4769 | 5 | - | - | ++ | ++ | ++ | + | ++ | ++ |
| L. ivanovii (ssp. ivanovii) | 3010 | ATCC 19119 | 5 | - | - | ++ | ++ | ++ | + | ++ | ++ |
| L. ivanovii (ssp. londoniensis) | 3060 | SLCC 3765 | 5 | - | - | ++ | (+) | - | + | ++ | - |
| L. innocua | 2011 | ATCC 33090 | 6a | - | - | ++ | (+) | - | + | + | - |
| L. innocua | 2012 | ATCC 33091 | 6b | - | - | ++ | ++ | ++ | + | ++ | ++ |
| L. welshimeri | 50146 | SLCC 7622 | 6a | - | - | ++ | ++ | + | + | (+) | - |
| L. grayi (ssp. grayi) | 6036 | ATCC 19120 | - | (+) | - | (+) | - | ++ | (+) | + | - |
| L. grayi (ssp. murrayi) | 6037 | ATCC 25401 | - | (+) | - | (+) | + | ++ | (+) | + | - |

## Claims

1. A polypeptide with an amino acid sequence according to SEQ ID NO:1 or polypeptide fragments thereof, having the amino acid sequence according to SEQ ID NO: 3 or 4, the polypeptide fragments having the property to bind the cell wall of Listeria, wherein the polypeptide fragments do not exhibit any enzymatically active cell wall hydrolysis regions anymore.

2. A nucleic acid molecule comprising a nucleotide sequence coding a polypeptide or a polypeptide fragment according to claim 1.

3. A vector comprising a nucleic acid molecule according to claim 2.

4. A host cell comprising a nucleic acid molecule according to claim 2 or a vector according to claim 4.

5. The polypeptide according to claim 1 for use as a human medical, veterinary medical or diagnostic substance, as an antimicrobial in food or on cosmetics, as disinfecting agent or in the environmental field.

6. The polypeptide for use according to claim 5, wherein the foods are dairy products, smoked fish, salted fish, frozen seafood, meat products, salads or convenience products.

7. The polypeptide according to claim 1 for the application as a human medical, veterinary medical or diagnostic substance for therapy and/or prevention of diseases caused by listeria or for the diagnosis of listeria contaminations.

8. The polypeptide for use according to claim 7, wherein the diseases caused by listeria comprise listeriosis, gastroenteritis, meningitis, encephalitis, sepsis, local wound infections caused by smear infections and inflammations of conjunctiva and cornea.

9. The polypeptide for use according to one of the claims 7 or 8 during prenatal care.

10. Use of the polypeptide according to claim 1 for the in vitro detection of listeria contaminations in medicine, in the food industry and food analysis, in livestock breeding, in drinking water analysis or environmental analysis.

11. Use of the polypeptide according to claim 1 as an antimicrobial substance in food or in cosmetics, as disinfecting agent or in the environmental field.

12. Use of the polypeptide according to claim 1 as an antimicrobial substance in food processing devices, in food processing facilities, on surface areas that come into contact with food, and in facilities that are used for storage or processing of food.

13. Use according to claim 12, wherein the antimicrobial agent is used in combination with other disinfecting agents, antibiotics and/or enzymes.

## Patentansprüche

1. Ein Polypeptid mit einer Aminosäuresequenz nach SEQ ID NO:1 oder Polypeptidfragmente davon mit der Aminosäuresequenz nach SEQ ID NO:3 oder 4, wobei die Polypeptidfragmente die Eigenschaft haben, die Zellwand von Listeria zu binden, wobei die Polypeptidfragmente keine enzymatisch aktive Zellwandhydrolyseregionen mehr aufweisen.

2. Ein Nukleinsäuremolekül enthaltend eine Nukleotidsequenz kodierend für ein Polypeptid oder ein Polypeptidfragment nach Anspruch 1.

3. Ein Vektor enthaltend ein Nukleinsäuremolekül nach Anspruch 2.

4. Eine Wirtszelle enthaltend ein Nukleinsäuremolekül nach Anspruch 2 oder einen Vektor nach Anspruch 4.

5. Das Polypeptid nach Anspruch 1 zur Verwendung als humanmedizinische, tiermedizinische oder diagnostische Substanz, als eine antimikrobielle in Lebensmitteln oder in der Kosmetik, als Desinfektionsmittel oder im Umweltbereich.

6. Das Polypeptid zur Verwendung nach Anspruch 5, wobei die Lebensmittel Milchprodukte, Räucherfisch, gepökelter Fisch, gefrorene Meeresfrüchte, Fleischprodukte, Salate oder Convenienceprodukte sind.

7. Das Polypeptid nach Anspruch 1 für die Anwendung als human medizinische, tiermedizinische oder diagnostische Substanz für die Therapie und/oder Vorbeugung von Krankheiten, die durch Listeria verursacht werden, oder für die Diagnose von Listeriakontaminationen.

8. Das Polypeptid zur Verwendung nach Anspruch 7, wobei die Krankheiten verursacht werden durch Listeriose, Gastroenteritis, Meningitis, Encephalitis, Sepsis, lokale Wundinfektionen verursacht durch Schmierinfektionen und Bindehaut- und Hornhautentzündungen.

9. Das Polypeptid zur Verwendung nach einem der Ansprüche 7 oder 8 während der Schwangerschaftsvorsorge.

10. Verwendung des Polypeptids nach Anspruch 1 zur *in vitro* Detektion von Listeriakontaminationen in der Medizin, in der Lebensmittelindustrie und Lebensmittelanalyse, in der Tierzucht, in der Trinkwasseranalyse oder Umweltanalyse.

11. Verwendung des Polypeptids nach Anspruch 1 als antimikrobielle Substanz in Lebensmitteln oder in der Kosmetik, als Desinfektionsmittel oder im Umweltbereich.

12. Verwendung des Polypeptids nach Anspruch 1 als antimikrobielle Substanz für Lebensmittelverarbeitungsgeräte, in Lebensmittelverarbeitungseinrichtungen, auf Oberflächenbereichen, die mit Lebensmitteln in Kontakt kommen, und in Einrichtungen, die für die Lagerung und Verarbeitung von Lebensmitteln verwendet werden.

13. Verwendung nach Anspruch 12, wobei das antimikrobielle Agens in Kombination mit anderen Desinfektionsmitteln, Antibiotika und/oder Enzymen verwendet wird.

## Revendications

1. Polypeptide avec une séquence d'acides aminés selon SEQ ID NO : 1 ou des fragments polypeptidiques de celui-ci, ayant la séquence d'acides aminés selon SEQ ID NO : 3 ou 4, les fragments polypeptidiques possédant la propriété de lier la paroi cellulaire de Listeria, où les fragments polypeptidiques ne présentent plus de régions enzymatiquement actives d'hydrolyse de la paroi cellulaire.

2. Molécule d'acide nucléique comprenant une séquence nucléotidique codant pour un polypeptide ou un fragment polypeptidique selon la revendication 1.

3. Vecteur comprenant une molécule d'acide nucléique selon la revendication 2.

4. Cellule hôte comprenant une molécule d'acide nucléique selon la revendication 2 ou un vecteur selon la revendication 4.

5. Polypeptide selon la revendication 1 pour une utilisation en tant que substance médicale humaine, médicale vétérinaire ou de diagnostic, en tant qu'antimicrobien dans les aliments ou sur les cosmétiques, en tant qu'agent désinfectant ou dans le domaine environnemental.

6. Polypeptide pour une utilisation selon la revendication 5, où les aliments sont des produits laitiers, du poisson fumé, du poisson salé, des fruits de mer congelés, des produits à base de viandes, des salades ou des produits prêts à consommer.

7. Polypeptide selon la revendication 1 pour l'application en tant que substance médicale humaine, médicale vétérinaire ou de diagnostic pour le traitement et/ou la prévention de maladies provoquées par la Listeria ou pour le diagnostic de contaminations par la Listeria.

8. Polypeptide pour une utilisation selon la revendication 7, où les maladies provoquées par la Listeria comprennent la listériose, une gastro-entérite, une méningite, une encéphalite, une septicémie, des infections de plaies locales provoquées par des infections de contact et des inflammations de la conjonctive et de la cornée.

9. Polypeptide pour une utilisation selon l'une des revendications 7 ou 8 durant les soins prénatals.

10. Utilisation du polypeptide selon la revendication 1 pour la détection *in vitro* de contaminations par la Listeria en médecine, dans l'industrie alimentaire et l'analyse des aliments, dans l'élevage du bétail, dans l'analyse de l'eau de boisson ou l'analyse environnementale.

11. Utilisation du polypeptide selon la revendication 1 en tant que substance antimicrobienne dans les aliments ou dans les cosmétiques, en tant qu'agent désinfectant ou dans le domaine environnemental.

12. Utilisation du polypeptide selon la revendication 1 en tant que substance antimicrobienne dans les dispositifs de traitement des aliments, dans les établissements de traitement des aliments, sur les surfaces qui entrent en contact avec des aliments, et dans les établissements qui sont utilisés pour le stockage ou le traitement des aliments.

13. Utilisation selon la revendication 12, où l'agent antimicrobien est utilisé en combinaison avec d'autres agents désinfectants, antibiotiques et/ou enzymes.


### SEQUENCE LISTING

<110> Profos AG
<120> NEW ENDOLYSIN POLYP40
<130> PRO-036 PCT
<140> unknown
   <141> 2009-07-24
<150> 10 2008 002 972.6
   <151> 2008-07-25
<160> 4
<170> PatentIn version 3.3
<210> 1
   <211> 344
   <212> PRT
   <213> Phage P40
<400> 1
   Met Val Leu Val Leu Asp Ile Ser Lys Trp Gln Pro Thr Val Asn Tyr
   1               5                   10                  15
   Ser Gly Leu Lys Glu Asp Val Gly Phe Val Val Ile Arg Ser Ser Asn
               20                  25                  30
   Gly Thr Gln Lys Tyr Asp Glu Arg Leu Glu Gln His Ala Lys Gly Leu
           35                  40                  45
   Asp Lys Val Gly Met Pro Phe Gly Leu Tyr His Tyr Ala Leu Phe Glu
       50                  55                  60
   Gly Gly Gln Asp Thr Ile Asn Glu Ala Asn Met Leu Val Ser Ala Tyr
   65                  70                  75                  80
   Lys Lys Cys Arg Gln Leu Gly Ala Glu Pro Thr Phe Leu Phe Leu Asp
                   85                  90                  95
   Tyr Glu Glu Val Lys Leu Lys Ser Gly Asn Val Val Asn Glu Cys Gln
               100                 105                 110
   Arg Phe Ile Asp His Val Lys Gly Gln Thr Gly Val Lys Val Gly Leu
           115                 120                 125
   Tyr Ala Gly Asp Ser Phe Trp Lys Thr His Asp Leu Asp Lys Val Lys
       130                 135                 140
   His Asp Leu Arg Trp Val Ala Arg Tyr Gly Val Asp Asn Gly Lys Pro
   145                 150                 155                 160
   Ser Thr Lys Pro Ser Ile Pro Tyr Asp Leu Trp Gln Tyr Thr Ser Lys
   165 170 175
   Gly Arg Ile Lys Ala Ile Ala Ser Pro Val Asp Met Asn Thr Cys Ser
   180 185 190
   Ser Asp Ile Leu Asn Lys Leu Lys Gly Ser Lys Ala Pro Val Lys Pro
   195 200 205
   Ala Pro Lys Pro Thr Pro Ser Lys Pro Ala Pro Ala Lys Pro Ala Pro
   210 215 220
   Lys Thr Thr Thr Lys Tyr Val Asn Thr Ala His Leu Asn Ile Arg Glu
   225 230 235 240
   Lys Ala Ser Ala Asp Ser Lys Val Leu Gly Val Leu Asp Leu Asn Asp
   245 250 255
   Ser Val Gln Val Ile Ser Glu Ser Gly Gly Trp Ser Lys Leu Lys Ser
   260 265 270
   Gly Asn Lys Gln Val Tyr Val Ser Ser Lys Tyr Leu Ser Lys Ser Lys
   275 280 285
   Thr Thr Pro Lys Ala Lys Pro Ser Ser Lys Gln Tyr Tyr Thr Ile Lys
   290 295 300
   Ser Gly Asp Asn Leu Ser Tyr Ile Ala Lys Lys Tyr Lys Thr Thr Val
   305 310 315 320
   Lys Gln Ile Gln Asn Trp Asn Gly Ile Lys Asp Ala Asn Lys Ile Tyr
   325 330 335
   Ala Gly Gln Lys Ile Arg Val Lys
   340
<210> 2
   <211> 1035
   <212> DNA
   <213> Phage P40
<400> 2
   atggtattag ttttagacat ttcaaaatgg caaccgacag tgaattattc aggactaaaa 60
   gaagatgtag gattcgttgt cattcgttct agcaacggaa cacagaagta tgatgagaga 120
   ttagagcaac acgcaaaagg cttagataaa gtgggaatgc ctttcggact gtaccactac 180
   gctttatttg aaggtggaca agatactatc aatgaagcga atatgttagt tagcgcatat 240
   aagaaatgtc gtcaattagg cgcagaacca acattcttgt tcttagatta tgaagaagtc    300
   aagttaaaat ctggtaatgt ggtaaacgaa tgtcagagat ttatagacca tgtgaaaggt    360
   caaactgggg tcaaagtagg actttatgct ggggatagtt tttggaagac gcacgattta    420
   gataaagtca agcacgattt aagatgggta gctagatatg gggtagataa cggtaaaccg     480
   tctacaaaac catctatacc ttatgatttg tggcagtata cttccaaggg gcgaattaaa    540
   gccattgctt cacctgtaga tatgaataca tgttctagcg acatattgaa caaattaaaa    600
   ggttcaaaag cacctgttaa accagcacca aaaccgacac ctagtaagcc agcaccagcg    660
   aaaccagcac caaaaacgac tactaaatat gtcaatacgg cacatttaaa tattcgtgaa    720
   aaggcaagtg ctgactcgaa agtattggga gttcttgacc tcaacgattc cgtacaggtc    780
   atttctgaat caggtggatg gtctaagttg aaatctggga acaagcaagt atatgtttct    840
   agcaagtatc ttagtaagtc aaaaacgaca ccgaaggcga aaccaagctc gaaacagtat    900
   tatactatta aaagcggtga taatttaagt tacattgcta agaagtataa aactacagta    960
   aaacagattc aaaactggaa cggtatcaag gatgctaaca aaatttacgc aggtcaaaaa   1020
   attagagtta aataa                                                    1035
<210> 3
   <211> 200
   <212> PRT
   <213> Phage P40
<400> 3
   Met Val Leu Val Leu Asp Ile Ser Lys Trp Gln Pro Thr Val Asn Tyr
   1               5                   10                  15
   Ser Gly Leu Lys Glu Asp Val Gly Phe Val Val Ile Arg Ser Ser Asn
               20                  25                  30
   Gly Thr Gln Lys Tyr Asp Glu Arg Leu Glu Gln His Ala Lys Gly Leu
           35                  40                  45
   Asp Lys Val Gly Met Pro Phe Gly Leu Tyr His Tyr Ala Leu Phe Glu
       50                  55                  60
   Gly Gly Gln Asp Thr Ile Asn Glu Ala Asn Met Leu Val Ser Ala Tyr
   65                  70                  75                  80
   Lys Lys Cys Arg Gln Leu Gly Ala Glu Pro Thr Phe Leu Phe Leu Asp
                   85                  90                  95
   Tyr Glu Glu Val Lys Leu Lys Ser Gly Asn Val Val Asn Glu Cys Gln
               100                 105                 110
   Arg Phe Ile Asp His Val Lys Gly Gln Thr Gly Val Lys Val Gly Leu
           115                 120                 125
   Tyr Ala Gly Asp Ser Phe Trp Lys Thr His Asp Leu Asp Lys Val Lys
       130                 135                 140
   His Asp Leu Arg Trp Val Ala Arg Tyr Gly Val Asp Asn Gly Lys Pro
   145                 150                 155                 160
   Ser Thr Lys Pro Ser Ile Pro Tyr Asp Leu Trp Gln Tyr Thr Ser Lys
                   165                 170                 175
   Gly Arg Ile Lys Ala Ile Ala Ser Pro Val Asp Met Asn Thr Cys Ser
               180                 185                 190
   Ser Asp Ile Leu Asn Lys Leu Lys
           195                 200
<210> 4
   <211> 118
   <212> PRT
   <213> Phage P40
<400> 4
   Thr Thr Lys Tyr Val Asn Thr Ala His Leu Asn Ile Arg Glu Lys Ala
   1               5                   10                  15
   Ser Ala Asp Ser Lys Val Leu Gly Val Leu Asp Leu Asn Asp Ser Val
               20                  25                  30
   Gln Val Ile Ser Glu Ser Gly Gly Trp Ser Lys Leu Lys Ser Gly Asn
           35                  40                  45
   Lys Gln Val Tyr Val Ser Ser Lys Tyr Leu Ser Lys Ser Lys Thr Thr
       50                  55                  60
   Pro Lys Ala Lys Pro Ser Ser Lys Gln Tyr Tyr Thr Ile Lys Ser Gly
   65                  70                  75                  80
   Asp Asn Leu Ser Tyr Ile Ala Lys Lys Tyr Lys Thr Thr Val Lys Gln
                   85                  90                  95
   Ile Gln Asn Trp Asn Gly Ile Lys Asp Ala Asn Lys Ile Tyr Ala Gly
               100                 105                 110
   Gln Lys Ile Arg Val Lys
           115